# EUROPÄISCHE PATENTSCHRIFT

(11) **EP 2 944 267 B1**
(45) Veröffentlichungstag und Bekanntmachung des Hinweises auf die Patenterteilung: **27.02.2019**
(21) Anmeldenummer: 14180303.1
(22) Anmeldetag: 08.08.2014
(51) Int. Cl.: A61B 17/04, A61B 17/88, A61B 90/00

(54) **Chirurgische Fadenzugvorrichtung**
Surgical suturing device
Dispositif de traction de fil chirurgical

(30) Priorität: 12.05.2014 EP 14167908
(43) Veröffentlichungstag der Anmeldung: 18.11.2015
(73) Patentinhaber: Mathys AG Bettlach, 2544 Bettlach (CH)
(72) Erfinder: Gyssler, Bernhard, 8800 Thalwil (CH); Gross, Christoph, 3027 Bern (CH); Häberli, Janosch, Dr., 4500 Solothurn (CH); Delfosse, Daniel, Dr., 3303 Jegenstorf (CH)
(74) Vertreter: Körfer, Thomas

(56) Entgegenhaltungen:
- EP-A1- 1 987 779
- EP-A1- 2 730 232
- EP-A2- 2 422 712
- US-A1- 2010 087 837

## Beschreibung

Die Erfindung bezieht sich auf eine chirurgische Fadenzugvorrichtung zum Anziehen und Spannen eines Fadens in chirurgischen Verfahren.

In chirurgischen Verfahren werden Fäden, die entweder aus einer einzelnen Faser oder einem Geflecht aus einer Vielzahl von Fasern bestehen, für eine Vielzahl von Anwendungen verwendet. Beispielsweise wird ein Faden zur Unterstützung eines gerissenen Kreuzbandes in ein Kniegelenk eingesetzt. Auch werden zur Sicherung von gebrochenen oder geschwächten Knochen sogenannten Cerclage Fäden verwendet mit deren Hilfe eine Knochenfixierung möglich ist. Dabei wird der Cerclage Faden bzw. Draht um den jeweiligen Knochen herumgeführt und gespannt. Des Weiteren finden Fäden in chirurgischen Verfahren zur Fixierung von Gewebematerial Verwendung. Der jeweilige Faden wird mit einer definierten Kraft angezogen und kann dann beispielsweise am Knochen oder an einem Gegenstück bzw. Implantat fixiert werden. Das Ziehen und Spannen des Fadens erfolgt im Allgemeinen mit einer dafür vorgesehenen Fadenzugvorrichtung.

Aus der US 2008/0275477 A1 ist eine Fadenzugvorrichtung bekannt, die ein kanüliertes Röhrchen, einen daran angeordneten axialen Griff mit einem Schlitz zur Durchführung eines Fadens und eine in den Schlitz des Griffes reichende Fadenhalterung umfasst. Der zu spannende Faden wird in das kanülierte Röhrchen eingeführt und weiter durch den Griff zum Fadenhalter geführt und dort fixiert. Durch Drehen der Einstellvorrichtung wird anschließend der Faden angezogen bzw. gespannt.

Ist der Faden mit der gewünschten Spannkraft gespannt kann dieser beispielsweise am Knochen, an einem vorgesehenen Gegenstück oder an einem Verankerungsimplantat fixiert werden. Da die Fixierung bei gespanntem Faden vorgenommen werden muss, ist der Zugang zum Knochen bzw. Gegenstück oftmals durch die Fadenzugvorrichtung verdeckt bzw. nur durch Verrücken der Fadenzugvorrichtung und einer eventuellen Änderung der am Faden wirkenden Spannung zugänglich. Bei der Fadenzugvorrichtung gemäß der US 2008/0275477 wird die Handhabung der Fadenzugvorrichtung des Weiteren durch die Anordnung des Fadenhalters in einem Schlitz des axialen Griffs zur Fadenführung erschwert.

Aus der nachveröffentlichten EP 2 730 232 A2 geht ebenfalls eine chirurgische Fadenspannvorrichtung zum Spannen eines an einem Implantat zu fixierenden Fadens mit einem rohrförmigen Schaft einer axialen Durchgangsöffnung und mit einem distalen Ende und einem proximalen Ende hervor. Die Fadenspannvorrichtung hat ferner ein Kontaktelement, das mit dem proximalen Ende des Schafts verbunden ist und eine Öffnung aufweist, die sich von dem dem Schaft abgewandten Ende des Kontaktelements in axialer Richtung teilweise durch das Kontaktelement erstreckt, und ein Spannelement mit einem Fadenhalter zum Fixieren des Fadens.

Bei der EP 1 987 779 A1 ist der Fadenhalter direkt im Inneren des Griffs der gezeigten Fadenspannvorrichtung angeordnet, welcher durch die äußere Griffhülle und ein darin geführtes Bauteil gebildet ist. Dieses Dokument zielt daher auf eine Anordnung des Fadenhalters direkt innerhalb des Griffs ab.
Bei der EP 2 422 712 A2 ist der Fadenhalter und dessen T-förmiger Griff seitlich des Griffs der Fadenzugvorrichtung angeordnet. Alle Figuren zeigen eine seitliche Anordnung des Haltestücks und der Fadenkupplung bezüglich des Griffs der Fadenzugvorrichtung.
Bei der US 2010/0087837 A1 ist sichtlich, dass die den Fadenhalter bildenden Löcher und der damit verbundene Halter innerhalb eines zylindrischen Griffs bzw. seitlich dazu angeordnet ist. Der Fadenhalter ist somit direkt innerhalb der den Griff bildenden Bauteil angeordnet.

Basierend auf dem bekannten Stand der Technik ist es daher die Aufgabe der Erfindung, eine chirurgische Fadenzugvorrichtung zu schaffen, die eine vereinfachte Handhabung beim Spannen des Fadens ermöglicht. Zudem soll ein einfacher und bequemer Zugang zum Knochen bzw. zu einem Gegenstück ermöglicht werden, um beispielsweise den Faden in exakt der eingestellten Spannung fixieren zu können.
Die Aufgabe wird durch die erfindungsgemäße chirurgische Fadenzugvorrichtung mit den Merkmalen des Anspruchs 1 gelöst.
Die erfindungsgemäße chirurgische Fadenzugvorrichtung zum Spannen eines an einem Implantat zu fixierenden Fadens umfasst einen sich in Längsrichtung der Fadenzugvorrichtung erstreckenden axialen Griff, einen mit dem axialen Griff verbundenen, vorzugsweise rohrförmigen, Schaft mit einem proximalen Ende und einem distalen Ende, eine durch den Griff und den Schaft in Längsrichtung der Fadenzugvorrichtung verlaufende Durchgangsöffnung, und ein Spannelement mit einem Fadenhalter zum Fixieren des Fadens. Der Fadenhalter ist auf einer dem Schaft gegenüberliegenden Seite des axialen Griffs angeordnet und ist über ein Halteelement mit dem Griff verbunden. Die Durchgangsöffnung erstreckt sich auch durch das Halteelement. Der Fadenhalter ist gegenüber dem Griff in Längsrichtung der Fadenzugvorrichtung versetzt, d.h. nicht seitlich neben dem Griff sondern innerhalb einer virtuellen Verlängerung des Griffs in Längsrichtung einer Draufsicht senkrecht zur Längsachse der Fadenzugvorrichtung und senkrecht zur Drehachse des Fadenhalters angeordnet. Der Fadenhalter kann bevorzugt radial außerhalb der Durchgangsöffnung angeordnet sein.
Die Handhabung wird für den Benutzer der Fadenzugvorrichtung erheblich erleichtert, da das Spannelement bzw. der Fadenhalter nicht am Griff sondern gegenüber dem Griff axial versetzt angeordnet ist. Der Benutzer kann somit den axialen Griff für ein sicheres Halten der Vorrichtung umgreifen und mit der anderen Hand die gewünschte Fadenspannung durch Drehen des Fadenhalters einstellen. Auf diese Weise wird eine kompakte und einfach zu bedienende Fadenzugvorrichtung bereitgestellt.
Ist die Fadenzugvorrichtung am Knochen oder einem Gegenstück bzw. Implantat angesetzt, wird über eine vorzugsweise am distalen Ende des bevorzugt rohrförmigen Schafts angeordnete Öffnung der Faden aufgenommen und bevorzugt außerhalb des rohrförmigen Schafts zu dem axial gegenüber dem Griff versetzten und bevorzugt radial außerhalb der Durchgangsöffnung angeordnet Fadenhalter geführt und dort fixiert. Der Faden kann alternativ auch wenigstens teilweise innerhalb der Durchgangsöffnung zum Fadenhalter geführt werden. Durch Drehen des Spannelements wird der Faden gespannt. Ein Werkzeug zum Fixieren des Fadens am Knochen oder einem Gegenstück bzw. Implantat kann nun durch die axiale Durchgangsöffnung im rohrförmigen Schaft und Griff zum Knochen oder Gegenstück geführt werden und dort den Faden z.B. durch Eindrehen einer Klemmschraube fixieren. Das Werkzeug kann somit unter Beibehaltung der Position der Fadenzugvorrichtung an den Knochen bzw. an ein Gegenstück herangeführt werden. Eine Änderung der Fadenspannung durch eine aus ihrer ursprünglichen Lage versetzten Fadenzugvorrichtung wird somit minimiert bzw. gänzlich ausgeschlossen.

Die Außenkontur des distalen Endes des rohrförmigen Schafts kann eine mehrkantige, insbesondere sechskantige, Kontur aufweisen. Dies ist insbesondere vorteilhaft, wenn ein vorhandenes Gegenstück eine Mehrkantinnenkontur aufweist. Die Fadenzugvorrichtung ist somit unbeweglich bezüglich einer Drehbewegung um ihre Längsachse als auch unbeweglich in Bezug auf eine axiale Bewegung zum bzw. weg vom Gegenstück, die durch eine Anschlagseite im Gegenstück gegeben ist, am Gegenstück fixiert. Somit ist ein Verrutschen oder Verdrehen der Spannvorrichtung während des Spannens ausgeschlossen.
Das distale Ende des rohrförmigen Schafts kann des Weiteren zur Aufnahme bzw. zum selektiven Verbindung mit einem Fußteil ausgeprägt sein. Ein derartiges Fußteil ist vorzugsweise derart geformt, dass es ein stabiles Positionieren des distalen Endes des rohrförmigen Schafts auf einem Gewebe oder am Knochen ermöglicht. Hierbei weist das Fußteil neben einem Koppelelement zur Verbindung mit dem distalen Ende des rohrförmigen Schafts eine passende Form auf. Des Weiteren kann das Fußteil sich seitlich von der Bohrung wegerstreckende Arme aufweisen, welche die Auflagefläche am Gewebe oder am Knochen vergrößern. Die Arme können dabei jeweils Positionierungshilfen wie beispielsweise einen Dorn aufweisen, mit Hilfe deren eine korrekte Positionierung des Fußteils an der Knochen- oder Gewebeoberfläche möglich ist.

Der Fadenhalter kann alternativ direkt an einer Stirnseite des Griffs angeordnet sein.

Die in Längsrichtung verlaufende Durchgangsöffnung durch die Fadenzugvorrichtung ist vorzugsweise an beiden Seiten offen. Ein Werkzeug zum Fixieren des Fadens am Knochen oder Gegenstück kann somit von einer Rückseite der Fadenzugvorrichtung durch die axiale Durchgangsöffnung im Halteelement, Griff und Schaft zum Implantat geführt werden, um dort den Faden z.B. durch Eindrehen einer Klemmschraube zu fixieren.
In einem vorteilhaften Ausführungsbeispiel ist der Fadenhalter zur Längsachse der Durchgangsöffnung senkrecht versetzt und in einer vordefinierten Entfernung zur Längsachse angeordnet. Dabei ist vorzugsweise eine Drehklemme oder Welle des Fadenhalters senkrecht zur Längsachse der Durchgangsöffnung angeordnet. Der Fadenhalter kann insbesondere in Seitenansicht der Fadenzugvorrichtung nach oben versetzt sein. Die Entfernung zwischen einer Drehachse einer Welle des Fadenhalters und der Längsachse der Durchgangsöffnung ist vorzugsweise größer als der Durchmesser der Welle oder Drehklemme des Fadenhalters, welche zur Aufnahme und zum Aufwickeln des Fadens dient.
Der Griff der Fadenzugvorrichtung weist vorzugsweise eine in Längsrichtung verlaufende Ausnehmung auf, die von einem Ansatzbereich des Schaftes in Längsrichtung auf den Fadenhalter bis zum gegenüberliegenden Ende des Griffs verläuft. Ein außerhalb des Schafts geführter Faden kann somit in der Ausnehmung des Griffs hin zum Fadenhalter geführt werden. Dies ermöglicht insbesondere ein sicheres und bequemes Halten der Fadenzugvorrichtung, ohne dass der Spannvorgang beispielsweise durch Einklemmen des Fadens zwischen Hand und Griff gestört wird.

Die Breite der Ausnehmung in Längsrichtung nimmt vorzugsweise in Richtung auf den Fadenhalter hin zu. Die Ausnehmung des Griffs kann insbesondere ein trichterförmiger Schlitz sein.

Die Ausnehmung weist vorzugsweise eine parallel zur Längsachse der Durchgangsöffnung angeordnete Bodenfläche sowie eine erste und zweite dazu senkrecht angeordnete Seitenwand auf.

In einer bevorzugten Ausführungsform sind die erste und zweite Seitenwand gegenüberliegend angeordnet und weisen bezüglich einer Längsachse der Durchgangsöffnung in Draufsicht auf die Fadenzugvorrichtung unterschiedliche Neigungswinkel auf. Hierbei weist vorzugsweise die zweite Seitenwand zu der Längsache der Durchgangsöffnung einen größeren Neigungswinkel auf, als die erste Seitenwand.

Die erste Seitenwand kann in Draufsicht weniger als 5°, bevorzugt weniger als 3°, besonders bevorzugt weniger als 2° zur Längsachse der Durchgangsöffnung geneigt angeordnet sein. Die zweite Seitenwand kann in Draufsicht mehr als 5°, bevorzugt mehr als 8°, besonders bevorzugt mehr als 12° zur Längsachse der Durchgangsöffnung geneigt angeordnet sein.

Das sich vom Griff gegebenenfalls weg erstreckende Halteelement kann am Griff fest angeordnet oder in Längsrichtung zu diesem beweglich angeordnet sein.

Im Fall eines beweglich angeordneten Halteelements ist das den Fadenhalter aufweisende Spannelement vorzugsweise an diesem Halteelement fixiert. Dabei kann eine Feder in einem Hohlraum im Griff angeordnet sein, die sich zwischen einer Anlagefläche des Halteelements und einer Anlagefläche im Griff abstützt. Die Feder kann koaxial mit der Längsachse der Durchgangsöffnung oder parallel dazu angeordnet sein. Das Halteelement weist vorteilhafterweise eine Skala auf, welche eine relative Aussage bezüglich der angelegten Spannkraft des Fadens ermöglicht. Wird die Feder im Griff durch Spannen des Fadens komprimiert, zeigt die Skala des Haltelements einen Wert an, welcher proportional zur Fadenspannkraft ist. Dadurch ist eine eingestellte Spannung des Fadens bequem ablesbar und durch Arretieren des Spannelements fixierbar. Dies ist insbesondere mit der Sechskantaußenkontur des distalen Endes des rohrförmigen Schafts von Vorteil, da durch den festen Sitz der Fadenzugvorrichtung im Gegenstück bzw. Verankerungsimplantat der Faden bei der eingestellten Spannung gehalten wird und gleichzeitig z.B. der Tibiaknochen eines behandelten Kniegelenks bewegt werden kann und somit die eingestellte Fadenspannung noch während der Operation überprüft werden kann.

Weiterhin ist es von Vorteil, wenn der Schaft und/oder der Griff eine Öffnung aufweist bzw. aufweisen, die in umfänglicher Richtung weniger als 180 Grad geöffnet ist. Die Öffnung reicht in bevorzugter Weise in radiale Richtung bis zur Durchgangsöffnung. Dadurch kann der Verlauf des Fadens sowie das Einführen des zusätzlichen Werkzeugs in Fadenzugvorrichtung visuell verfolgt und überprüft werden. Somit können Unregelmäßigkeiten zeitnah erkannt und verhindert werden.

Von Vorteil ist es, wenn der Fadenhalter in Form einer Drehklemme ausgebildet ist. Eine Drehklemme weist z.B. eine oder zwei Backen auf, die jeweils um eine Achse drehbar sind und eine Bewegung des Fadens in Richtung der Zugspannung ermöglichen, eine Bewegung des Fadens in die entgegengesetzte Richtung aber verhindern. Somit kann der Faden einfach in eine Drehklemme eingeführt und ein Verlieren des Fadens bei Zug in die Gegenrichtung verhindert werden.

Ebenso weist das Spannelement eine sattelförmige Halterung auf, welche vorzugsweise fest mit dem Griff und/oder Halteelement der Fadenzugvorrichtung verbunden ist. Die sattelförmige Halterung kann ebenso lösbar mit dem Griff und/oder dem Halteelement verbunden sein.

Das Spannelement selbst umfasst zusätzlich eine Spanneinheit, die eine Fadenspule mit dem Fadenhalter und ein Gesperre mit einem Drehgriff umfasst. Der Fadenhalter bildet vorteilhafterweise zumindest einen Teil einer Achse der Fadenspule aus. Somit kann der im Fadenhalter fixierte Faden durch Drehen des Drehgriffs auf die Achse der Fadenspule aufgewickelt werden. Das Gesperre ermöglicht es, den Faden mit der eingestellten Spannung zu halten, ohne dass ein Operateur bzw. Benutzer den Drehgriff festhalten muss. Dies ermöglicht in einfacher Weise eine Kontrolle der Fadenspannung am z.B. operierten Band (Ligamentum) ohne die Fadenzugvorrichtung aktiv in der eingestellten Spannung halten zu müssen.

Die Fadenzugvorrichtung kann einen Einführtrichter aufweisen, welcher eingangsseitig an der Durchgangsöffnung angeordnet ist. Dies ermöglicht eine vereinfachte Einführung eines Werkzeugs in die Durchgangsöffnung.
Der Fadenhalter der Fadenzugvorrichtung ist vorzugsweise ausgeprägt mit Hilfe eines AO (Arbeitsgemeinschaft für Osteosynthese) Drehmomenten-Schlüssels kontaktiert und betätigt zu werden. Dabei weist der Fadenhalter vorzugsweise eine Welle auf, auf welche mit Hilfe des Drehmomenten-Schlüssels ein vordefiniertes Drehmoment zur Einstellung einer definierten Fadenspannkraft übertragen werden kann.

Die Fadenzugvorrichtung kann eine Bodenplatte zum Auffangen einer sich vom Werkzeug gelösten Klemmschraube aufweisen. Eine derartige Bodenplatte ist in Einfuhrrichtung des Werkzeugs vorzugsweise hinter dem Einführtrichter angeordnet. Die Bodenplatte ist in Seitenansicht der Fadenzugvorrichtung vorzugsweise unterhalb des Fadenhalters angeordnet.
Das Spannelement der Fadenzugvorrichtung weist vorzugsweise Spüllöcher auf. Ausführungsbeispiele der erfindungsgemäßen chirurgischen Fadenzugvorrichtung sind in den Zeichnungen beispielhaft dargestellt und werden anhand der nachfolgenden Beschreibung näher erläutert. Es zeigen:
- Fig. 1: ein erstes Ausführungsbeispiel einer erfindungsgemäßen Fadenzugvorrichtung mit Griff in perspektivischer Ansicht;
- Fig. 2: das Ausführungsbeispiel der erfindungsgemäßen Fadenzugvorrichtung aus Fig. 1 als Schnittansicht durch eine Längsachse;
- Fig. 3: das Ausführungsbeispiel der erfindungsgemäßen Fadenzugvorrichtung aus Fig. 2 mit einer Spannungsskala in Draufsicht auf eine Öffnung in einem Griff bzw. Schaft;
- Fig. 4: ein Ausführungsbeispiel einer sattelförmigen Halterung einer Fadenzugvorrichtung in Draufsicht;
- Fig. 5: ein Ausführungsbeispiel einer Spanneinheit in Draufsicht;
- Fig. 6: eine vergrößerte Draufsicht auf den Griff und die darin angeordnete Ausnehmung gemäß dem ersten Ausführungsbeispiel der erfindungsmäßen Fadenzugvorrichtung;
- Fig. 7: eine Draufsicht auf die erfindungsgemäße Fadenzugvorrichtung gemäß dem ersten Ausführungsbeispiel;
- Fig. 8: eine Rückansicht der erfindungsgemäßen Fadenzugvorrichtung gemäß dem ersten Ausführungsbeispiel;
- Fig. 9a: eine vergrößerte perspektivische Darstellung des Fadenhalters der erfindungsgemäßen Fadenzugvorrichtung gemäß Fig. 1, und
- Fig. 9b: eine Draufsicht der Fadenzugvorrichtung gemäß Fig. 1 ohne den Fadenhalter.

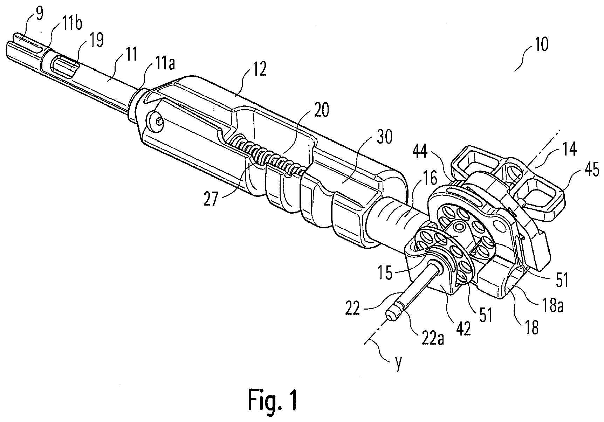

Einander entsprechende Teile sind in allen Figuren mit den gleichen Bezugszeichen versehen. Anhand von Fig. 1 wird nun der generelle Aufbau der Fadenzugvorrichtung erläutert und der Fadenverlauf beschrieben.

Die Fadenzugvorrichtung 10 umfasst einen axialen Griff 12, und einen sich davon erstreckenden Schaft 11. Der Schaft 11 ist vorzugsweise rohrförmig und weist ein proximales Ende 11a auf, welches mit dem Griff 12 verbunden ist, sowie ein distales Ende 11b. Der Schaft 11 weist eine Öffnung 9 auf, die sich vom distalen Ende axial in Richtung des Griffs 12 erstreckt. Die Öffnung ist vorzugsweise ein Schlitz welcher sich radial bis zu einer Durchgangsöffnung 18 durch den Schaft 11 erstreckt.

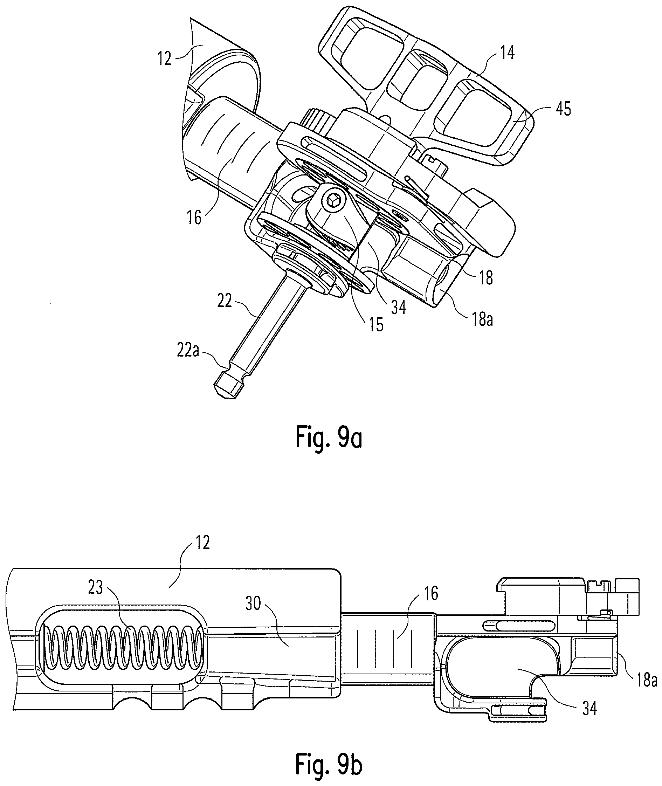

Der Schaft 11 weist eine in axialer Richtung, auch als Längsrichtung bezeichnet, durch die gesamte Länge des Schafts 11 verlaufende Durchgangsöffnung 18 auf. Die Durchgangsöffnung 18 erstreckt sich vorzugsweise durch die gesamte Fadenzugvorrichtung 10. An einem dem distalen Ende 11b des Schafts 11 gegenüberliegenden Öffnung der Durchgangsöffnung 18 ist vorzugsweise ein Einführtrichter 18a angeordnet (siehe auch Figur 9). Dieser erleichtert das Einführen von Werkzeug von einem rückwärtigen Ende der Fadenzugvorrichtung.

Die Fadenzugvorrichtung umfasst weiterhin ein Spannelement 14, welches in axialer Richtung beabstandet vom Griff angeordnet ist. Dies bedeutet, dass das Spannelement 14 nicht unmittelbar seitlich neben dem Griff sondern in einer axialen Verlängerung des Griffs 12 angeordnet ist.

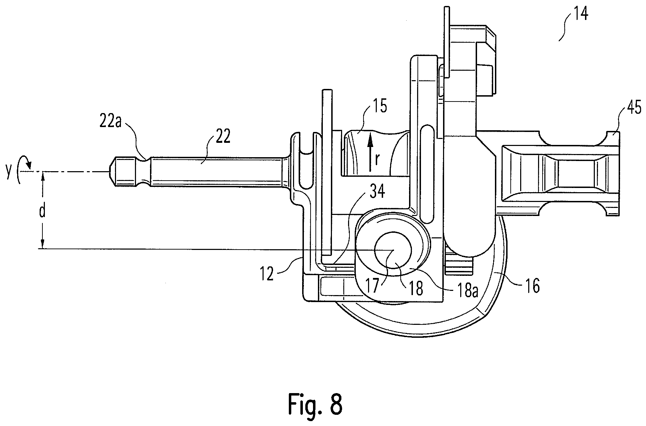

Vorzugsweise ist das Spannelement 14 auf einer dem Schaft 11 gegenüberliegenden Seite des Griffs 12 angeordnet. Des Weiteren ist das Spannelement vorzugsweise radial außerhalb der Durchgangsöffnung 18 durch die Fadenzugvorrichtung angeordnet. Auf diese Weise wird eine ungewünschte Überschneidung des Fadenverlaufs mit der Durchgangsöffnung oder einem darin eingeführten Werkzeug vermieden.
Das Spannelement 14 weist einen Fadenhalter 15 auf, welcher um eine Achse Y drehbar angeordnet ist. Die Drehachse Y des Spannelements 14 bzw. des Fadenhalters 15 ist vorzugsweise senkrecht versetzt zu einer Längsachse 17 der Durchgangsöffnung 18 angeordnet. Insbesondere kann die Drehachse Y in einer vordefinierten Entfernung d (siehe Figur 8) senkrecht und seitlich zur Längsachse 17 versetzt angeordnet sein. In Seitenansicht bzw. Rückansicht der Fadenzugvorrichtung 10 (siehe auch Figur 8) ist die Drehachse Y nach oben versetzt angeordnet.

Der Fadenhalter 15 ist mit einem Gesperre 44 und einem Drehgriff 45 des Spannelements 14 verbunden. Ein zu fixierender Faden, der hier der Übersicht halber nicht dargestellt ist, wird über die Öffnung 9 aus dem distalen Ende 11b des Schafts 11 herausgeführt und außerhalb des Schafts 11 in Längsrichtung zum Fadenhalter 15 geführt und dort fixiert. Der Faden läuft initial somit zur Längsachse 17 der Durchgangsöffnung 18 in Längsrichtung geneigt und mit dem Aufwickeln parallel zur Achse. Durch Drehen des Fadenhalters 15 bzw. des Drehgriffs 45, der über ein Gesperre 44 mit dem Fadenhalter 15 verbunden ist, wird über eine Drehbewegung der Faden auf den Fadenhalter 15 aufgewickelt und damit gespannt.

Der Fadenhalter 15 ist vorzugsweise mit Hilfe eines Haltelements 16 und/oder eine Halterung 42 am Griff 12 befestigt. Das Haltelement 16 ist vorzugsweise ein sich axial vom Griff 12 weg erstreckendes kanalförmiges Element, welches koaxial mit dem Schaft 11 angeordnet sein kann. Die Durchgangsöffnung 18 erstreckt sich somit auch durch das Haltelement 16. Eine optional zusätzlich vorgesehene separate Halterung 42 kann fest oder selektiv verbindbar mit dem Haltelement 16 ausgebildet sein. Das Haltelement 16 und/oder die Halterung 42 kann derart ausgeführt sein, dass eine Einstellbarkeit der Entfernung zwischen Drehachse Y des Fadenhalters 15 und der Längsachse 17 der Durchgangsöffnung 18 ermöglicht wird.

Der axiale Griff 12 weist eine greifbare Form, beispielsweise eine zylindrische oder im Wesentlichen zylindrische Form auf. Eine Rotationsachse des Griffs 12 kann koaxial oder parallel mit der Längsachse 17 durch die Durchgangsöffnung 18 angeordnet sein. Die Länge und der Durchmesser des Griffs 12 sind vorzugsweise derart gewählt, dass ein Benutzer den Griff mit der ganzen Hand umgreifen kann.

Der Griff 12 weist ebenfalls eine in axialer Richtung durch die gesamte Länge des Griffs 12 führende Durchgangsöffnung 18 auf. Somit erstreckt sich eine Durchführungsöffnung 18 vom distalen Ende 11b des Schafts über den Schaft 11, den Griff 12 und das Haltelement 16 durch die gesamte Länge der Fadenzugvorrichtung 10.

Der Griff 12 ist bevorzugt aus Kunststoff oder Metall, beispielsweise Aluminium, gefertigt.

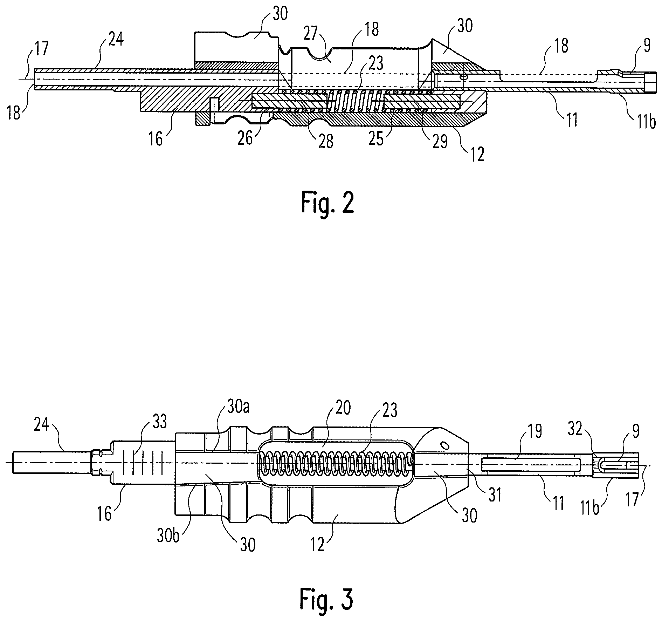

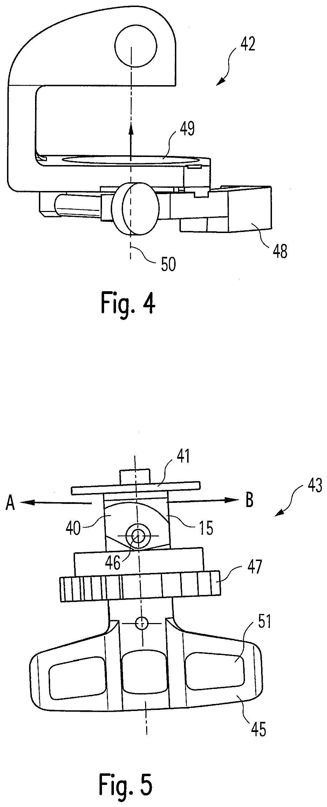

Der Griff 12 weist eine Ausnehmung 30 auf, die in axialer Richtung durch den Griff 12 verläuft. Die Ausnehmung 30 ist vorzugsweise in einer Mantelfläche des Griffs geformt. Die Ausnehmung 30 ist derart angeordnet, dass ein Faden von der Öffnung 9 des Schafts hin zum Fadenhalter 15 in ihr geführt werden kann. Hierzu führt die Ausnehmung von einem Ansatzbereich 31 des Schafts 11 (siehe Figur 4) in axialer Richtung zum genüberliegenden Ende des Griffs 12 und tritt dort in Richtung auf den Fadenhalter 15 aus, sodass der Faden gerade von der Öffnung 9 des Schafts 11 zum Fadenhalter 15 verläuft. Die Ausnehmung 30 ist vorzugsweise ein trichterförmiger Schlitz, welcher im Wesentlichen parallel zur Längsachse 17 der Durchgangsöffnung 18 angeordnet ist. Die Tiefe der Ausnehmung 30 im Griff 12 ist derart gewählt, dass die Ausnehmung 30 sich vorzugsweise nicht bis zur Durchgangsöffnung 18 erstreckt.
Der Schaft 11 sowie optional auch der Griff 12 weisen eine Öffnung 19 bzw. 20 auf, die in umfänglicher Richtung einen Winkel von weniger als 180 Grad einnimmt und bis zur Durchführungsöffnung 18 reicht. Die Öffnung 19 bzw. 20 erlaubt somit einen direkten Blickkontakt in die Durchgangsöffnung 18. Dadurch kann ein Benutzer bzw. Operateur den Fadenverlauf und das Einführen des zusätzlichen Werkzeugs in die Durchgangsöffnung 18 kontinuierlich beobachten und kontrollieren. In einer alternativen Ausführungsform kann sich die in Figur 1 gezeigte Öffnung 19 im Schaft 11 im Wesentlichen über die gesamte Länge des Schafts 11 bis hin zum Ansatzbereich 31 (siehe Figur 3) des Schafts 11 bzw. des Griffs 12 erstrecken. Ebenso ist es möglich, dass sich die Öffnung 20 des Griffs 12 in Wesentlichen durch den ganzen Griff 12 erstreckt.

Das Spannelement 14 weist vorzugsweise Spüllöcher 51 auf. Fig. 2 zeigt einen axialen Schnitt durch die in Fig. 1 dargestellte Fadenzugvorrichtung 10, wobei das Spannelement 14 in dieser Figur zur Vereinfachung nicht dargestellt ist. Es ist deutlich die Durchgangsöffnung 18 zu erkennen, die vom distalen Ende 11b durch den Schaft 11 und den Griff 12 hindurch ausgebildet ist. Wie gezeigt, weist gemäß diesem Ausführungsbeispiel das Haltelement 16 ein rohrförmigen Element 24 auf. Der hohle Innenraum des Haltelements 16 bzw. des rohrförmigen Elements 24 bildet dabei eine Weiterführung der Durchgangsöffnung 18 durch die Fadenzugvorrichtung 10, siehe Fig. 2, aus.

In einem Hohlraum 27 des Griffs 12 ist eine Feder 23 eingebracht, die sich an einer Anlagefläche 26 des Halteelements 16 und eine Anlagefläche 25 im Griff 12 bzw. am Schaft 11 abstützt. Über die Anlagefläche 26 ragt ein erster Führungsstift 28 hervor. In gleicher Weise ragt ein zweiter Führungsstift 29 über die Anlagefläche 25 im Griff 12 hervor. Die Feder 23 übergreift den ersten und zweiten Führungsstift. Über den Abstand zwischen den zueinander gerichteten Enden des ersten und zweiten Führungsstiftes 28, 29 kann ein maximaler Kompressionsweg der Feder 23 und somit eine maximale Spannung des Fadens vorgegeben werden. Der erste und zweite Führungsstift 28 und 29 greifen jeweils in die z.B. als Spiralfeder ausgebildete Feder 23 ein und verhindern somit ein Verlieren oder Ausspringen der Feder 23 aus dem Griff 12. Wie in der Figur 2 gezeigt, kann die Feder 23 parallel zur Längsachse 17 angeordnet sein. Die Feder 23 kann ebenso koaxial mit der Längsachse 17 angeordnet sein.

Das Spannelement 14 ist dabei, wie in Fig. 1 dargestellt, am Haltelement 16 bzw. 24 unbeweglich oder beweglich in axialer Richtung fixiert. Wird nun der Faden durch Drehen des Drehgriffs 45 gespannt, wird das Haltelement 16 und das rohrförmige Element 24 entsprechend der wirkenden Kraft und Federkonstante der Feder 23 zum Schaft 11 hin verschoben. Über eine Skala 33, siehe Fig. 3, die am Halteelement 16 angebracht ist, kann dabei eine relative Spannkraft abgelesen werden.

Fig. 3 zeigt das Ausführungsbeispiel der Fadenzugvorrichtung aus Fig. 2 bzw. Fig. 1 in Draufsicht, ohne das Spannelement 14. Dabei ist deutlich die Öffnung 20 im Griff 12 sowie die Öffnung 19 im Schaft 11 und die Öffnung 9 im distalen Ende 11b sichtbar. Die Achse 17 bildet hier eine gemeinsame Achse der Durchgangsöffnung 18 vom distalen Ende 11b bis zum Halteelement 16 bzw. rohrförmigen Element 24 aus.

Das distale Ende 11b kann eine sechskantige Außenkontur 32 aufweisen. Die Außenkontur 32 kann auch rund ausgeführt sein. Ebenfalls ist die Ausnehmung 30, welche in Längsrichtung vom Schaft-seitigen Ende 31 des Griffs 11 bis zum gegenüberliegenden Ende des Griffs 12 verläuft, dargestellt.

Fig. 4 zeigt die sattelförmige Halterung 42 der Spannvorrichtung 14, mit der die Spannvorrichtung 14 am Schaft 11 bzw. am Haltelement 16 fix oder am rohrförmigen Element 24 lösbar befestigt ist. In eine Einschuböffnung 49 wird in Pfeilrichtung 50 eine Spanneinheit 43 eingeschoben und fixiert. Die Spanneinheit 43 kann dabei lösbar mit der sattelförmigen Halterung 42 verbunden sein.

Die Spanneinheit 43, siehe Fig. 5, umfasst eine Fadenspule 41, deren Achse zumindest teilweise von dem Fadenhalter 15 gebildet wird. Fest mit der Fadenspule 41 sind ein Sperrrad 47 sowie der Drehgriff 45 verbunden. Befindet sich die Spanneinheit 43 in der sattelförmigen Halterung 42, so liegt die Sperrklinke 48 auf dem Sperrrad 47 auf und bildet somit das Gesperre 44 (siehe Figur 1), welches ein Drehen in Spannrichtung des Sperrrads 45 erlaubt. In entgegengesetzter Richtung greift die Sperrklinke 48 in einen Zacken des Sperrrads 47 ein und blockiert die Drehung der Spanneinheit 43.

Der Fadenhalter 15 selber ist hier beispielsweise durch eine einbackige Drehklemme 40 ausgebildet. Diese Drehklemme 40 ist drehbar um die Achse 46 gelagert. Ein Faden kann dabei ohne signifikanten Widerstand in Pfeilrichtung A durch die Fadenhalterung gezogen werden. Bei einem Zug auf den Faden in die entgegengesetzte Richtung wird die Drehklemme 40 an die seitliche Wand der Fadenspule 41 gepresst und klemmt somit den Faden ein. Somit ist der Faden insbesondere unter einer Spannkraft in Pfeilrichtung B fixiert. Die Achse 46 der Drehklemme 40 des Fadenhalters 15 ist vorzugsweise senkrecht zur Längsachse 17 der Durchgangsöffnung 18 angeordnet.

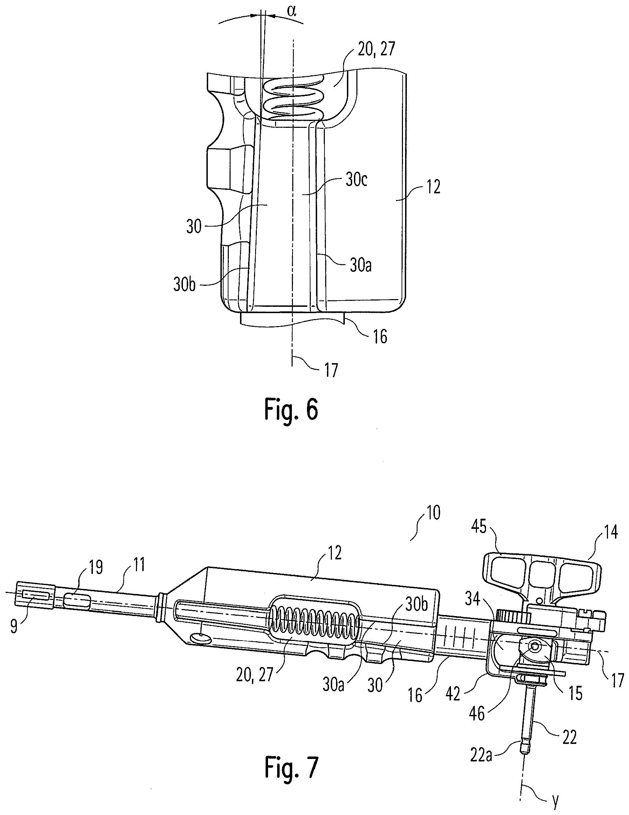

Fig. 6 zeigt eine Draufsicht auf die Ausnehmung 30 des Griffs 12 der Fadenzugvorrichtung gemäß dem ersten Ausführungsbeispiel der Erfindung. Die Ausnehmung weist eine parallel zur Längsachse der Durchgangsöffnung angeordnete Bodenfläche 30c sowie eine erste und zweite dazu senkrecht angeordnete Seitenwand 30a,30b auf. Die Ausnehmung ist im Wesentlichen trichterförmig in Draufsicht. Die Breite der Ausnehmung vergrößert sich dabei in Richtung auf den Fadenhalter 15. Die Breite der Ausnehmung bzw. der Abstand zwischen der ersten und zweiten Seitenwand 30a,30b beträgt an dem Schaft-seitigen Ende des Griffs 12 vorzugsweise zwischen 2mm und 8mm, an dem Fadenhalter-seitigen Ende vorzugsweise zwischen 5 und 15mm.

Die Seitenwände 30a,30b sind gegenüberliegend angeordnet und weisen bezüglich einer Längsachse der Durchgangsöffnung in Draufsicht auf die Fadenzugvorrichtung 10 unterschiedliche Neigungswinkel auf. Die zweite Seitenwand 30b weist in Draufsicht bezüglich der Längsache 17 der Durchgangsöffnung 18 einen größeren Neigungswinkel α auf, als die erste Seitenwand 30a.

Insbesondere kann die zweite Seitenwand 30b in Draufsicht mehr als 5°, bevorzugt mehr als 8°, besonders bevorzugt mehr als 12° zur Längsachse der Durchgangsöffnung geneigt angeordnet sein (siehe Winkel α in Fig. 7). Die erste Seitenwand kann in Draufsicht weniger als 5°, bevorzugt weniger als 3°, besonders bevorzugt weniger als 2° zur Längsachse der Durchgangsöffnung geneigt angeordnet sein.

Die Bodenfläche 30c sowie die erste und zweite Seitenwand 30a,30b der Ausnehmung sind vorzugsweise als kontinuierliche Oberflächen ausgeprägt. Die Bodenfläche 30c kann optional einen oder mehrere Öffnungen aufweisen, welche in ihrer Ausrichtung der Öffnung 20 des Griffs 12 entsprechen und somit einen Einblick in die Durchgangsöffnung 18 der Fadenzugvorrichtung gewähren.

Fig. 7 zeigt eine Draufsicht auf die erfindungsgemäße Fadenzugvorrichtung gemäß dem ersten Ausführungsbeispiel. Wie der Figur entnehmbar ist, liegt der Fadenhalter 15 in Draufsicht im Wesentlichen in der axialen Verlängerung des Griffs 12. Dabei ist der Fadenhalter 15 mit dem Griff mit Hilfe des Haltelements 16 fest verbunden.
Die Drehachse Y des Spannelements 14 ist senkrecht zur Längsachse 17 der Durchgangsöffnung angeordnet.
Der Fadenhalter 15 ist derart in axialer Verlängerung des Griffs 12 angeordnet, dass in Draufsicht auf die Fadenzugvorrichtung der Faden im Wesentlichen parallel zur Durchgangsöffnung 18 geführt wird.
Fig. 8 zeigt eine Rückansicht der erfindungsgemäßen Fadenzugvorrichtung gemäß dem ersten Ausführungsbeispiel.
Wie aus Fig. 8 entnehmbar weist die rückseitige Eintrittsöffnung der Durchgangsöffnung 18 einen Einführtrichter 18a zur vereinfachten Einfuhr eines Werkzeugs auf.
Die Drehachse Y des Spannelements 14 ist wie dargestellt vorzugsweise senkrecht zur Längsachse 17 der Durchgangsöffnung angeordnet. Der Fadenhalter 15 liegt radial außerhalb der Durchgangsöffnung 18. Die Drehachse Y des Spannelements 14 ist in einer vordefinierten Entfernung d zur Längsachse 17 der Durchgangsöffnung 18 angeordnet. Dabei ist die Entfernung d vorzugsweise größer oder gleich dem Durchmesser bzw. doppelten Radius r einer Welle des Fadenhalters 15, welche zur Aufnahme und zum Aufwickeln des Fadens dient.

Koaxial mit der Drehachse Y erstreckt sich vom Fadenhalter 15 eine Welle des Spannelements 14. Die Welle 22 ist vorzugsweise direkt mit dem Fadenhalter 15 verbunden und weist an ihrem distalen Ende eine nutartige Vertiefung 22a auf. Die Welle 22 ist ausgeprägt, von einem Drehmomenten-Schlüssel, vorzugsweise einem AO (Arbeitsgemeinschaft für Osteosynthese) Drehmomenten-Schlüssel kontaktiert zu werden. Ein Drehmomenten-Schlüssel kann somit auf das distale Ende der Welle 22 aufgesteckt werden. Durch Drehen des Drehmomenten-Schlüssels kann ein vom Schlüssel vordefiniertes Drehmoment auf die Welle 22 übertragen werden, wodurch eine spezifische Fadenspannung erzielt wird. Vorzugsweise wird mit Hilfe des Drehmomenten-Schlüssels ein gewünschtes Drehmoment eingestellt.

Wie in Figuren 9a und 9b gezeigt, ist der Einführtrichter 18a koaxial mit der Durchgangsöffnung 18 und bezüglich des Griffs 12 axial nach hinten versetzt angeordnet. Zwischen dem Einführtrichter 18a und dem Halteelement 16 bzw. Griff 12 ist eine Bodenplatte 34 angeordnet, welche zum Auffangen einer sich vom Werkzeug gelösten Klemmschraube ausgeprägt ist. Die Bodenplatte 34 ist vorzugsweise unterhalb des Fadenhalters 15 angeordnet. Die Bodenplatte 34 kann tangential zur Durchgangsöffnung 18 angeordnet sein oder radial außerhalb der Durchgangsöffnung.

Alle beschriebenen und/oder gezeigten Merkmale können im Rahmen der Erfindung vorteilhaft miteinander kombiniert werden. Die Erfindung ist nicht auf die beschriebenen Ausführungsbeispiele beschränkt.

## Patentansprüche

1. Chirurgische Fadenzugvorrichtung aufweisend
einen sich in Längsrichtung der Fadenzugvorrichtung erstreckenden axialen Griff (12),
einen mit dem axialen Griff verbundenen Schaft (11),
eine durch den Griff (12) und den Schaft (11) in Längsrichtung der Fadenzugvorrichtung verlaufende Durchgangsöffnung (18) und
ein Spannelement (14) mit einem Fadenhalter (15) zum Fixieren des Fadens,
wobei der Fadenhalter (15) auf einer dem Schaft (11) gegenüberliegenden Seite des axialen Griffs (12) angeordnet ist,
wobei der Fadenhalter (15) über ein Halteelement (16) mit dem Griff (12) verbunden ist und sich die Durchgangsöffnung (18) durch das Halteelement (16) erstreckt, und
wobei der Fadenhalter (15) gegenüber dem Griff (12) in Längsrichtung der Fadenzugvorrichtung versetzt ist, d.h. nicht seitlich neben dem Griff (12) sondern innerhalb einer virtuellen Verlängerung des Griffs (12) in Längsrichtung in einer Draufsicht senkrecht zur Längsachse der Fadenzugvorrichtung und senkrecht zur Drehachse (y) des Fadenhalters (15) angeordnet ist.

2. Vorrichtung nach Anspruch 1,
**dadurch gekennzeichnet,**
**dass** der Fadenhalter (15) radial außerhalb der Durchgangsöffnung (18) angeordnet ist.

3. Vorrichtung nach einem der Ansprüche 1 oder 2,
**dadurch gekennzeichnet,**
**dass** der Fadenhalter (15) zur Längsachse (17) der Durchgangsöffnung (18) senkrecht versetzt in einer vordefinierten Entfernung (d) angeordnet ist.

4. Vorrichtung nach einem der vorhergehenden Ansprüche,
**dadurch gekennzeichnet,**
**dass** der Fadenhalter (15) ausgeprägt ist mit Hilfe eines Drehmomenten-Schlüssels betätigt zu werden.

5. Vorrichtung nach einem der vorhergehenden Ansprüche,
**dadurch gekennzeichnet,**
**dass** der axiale Griff (12) eine in Längsrichtung verlaufende Ausnehmung (30) aufweist, die von einem Ansatzbereich (31) des Schaftes (11) in Längsrichtung auf den Fadenhalter (15) bis zum gegenüberliegenden Ende des Griffs (12) verläuft.

6. Vorrichtung nach Anspruch 5,
**dadurch gekennzeichnet,**
**dass** die Breite der Ausnehmung (30) in Längsrichtung auf den Fadenhalter (15) zunimmt.

7. Vorrichtung nach Anspruch 5 oder 6,
**dadurch gekennzeichnet,**
**dass** die Ausnehmung (30) eine erste und eine zweite, im Wesentlichen in Längsrichtung verlaufende, Seitenwand (30a, 30b) aufweist, welche sich gegenüber liegen, und dass die zweite Seitenwand (30b) bezüglich einer Längsache (17) der Durchgangsöffnung (18) einen größeren Neigungswinkel aufweist, als die erste Seitenwand (30a).

8. Vorrichtung nach einem der vorhergehenden Ansprüche,
**dadurch gekennzeichnet,**
**dass** der Griff (12) und/oder der Schaft (11) eine Öffnung (19, 20) aufweist, die bis zur Durchgangsöffnung (18) reicht und in umfänglicher Richtung weniger als 180° geöffnet ist.

9. Vorrichtung nach einem der vorhergehenden Ansprüche,
**dadurch gekennzeichnet,**
**dass** der Fadenhalter (15) in Form einer Drehklemme (40) ausgebildet ist.

## Claims

1. Surgical filament tensioning device comprising
an axial handle (12) extending in the longitudinal direction of the tensioning device,
a shaft (11) connected with the axial handle,
a through opening (18) running through the handle (12) and the shaft (11) in the longitudinal direction of the tensioning device and
a tensioning element (14) with a filament holder (15) for fixing the filament,
wherein the filament holder (15) is arranged on the opposite side of the axial handle (12) to the shaft (11), wherein the filament holder (15) is connected with the handle (12) by means of a retaining element (16) and
the through opening (18) extends through the retaining element (16), and
wherein the filament holder (15) is offset in relation to the handle (12) in the longitudinal direction of the tensioning device, i.e. is arranged not laterally next to the handle (12) but within a virtual extension of the handle (12) in the longitudinal direction in a plan view perpendicular to the longitudinal axis of the tensioning device and perpendicular to the axis of rotation (y) of the filament holder (15).

2. Device according to claim 1,
**characterised in that**
the filament holder (15) is arranged radially outside the through opening (18).

3. Device according to one of claims 1 or 2,
**characterised in that**
the filament holder (15) is arranged offset perpendicularly to the longitudinal axis (17) of the through opening (18) at a predefined distance (d).

4. Device according to one of the preceding claims,
**characterised in that**
the filament holder (15) is configured in order to be operated with the aid of a torque key.

5. Device according to one of the preceding claims,
**characterised in that**
the axial handle (12) comprises a recess (30) which runs in the longitudinal direction and extends from an attachment region (31) of the shaft (11) in the longitudinal direction to the filament holder (15) as far as the opposite end of the handle (12).

6. Device according to claim 5,
**characterised in that**
the breadth of the recess (30) increases in the longitudinal direction to the filament holder (15).

7. Device according to claim 5 or 6,
**characterised in that**
the recess (30) comprises a first and a second side wall (30a, 30b) which essentially run in the longitudinal direction and face one another, and
**in that** the second side wall (30b) has a greater angle of inclination than the first side wall (30a) with respect to a longitudinal axis (17) of the through opening (18).

8. Device according to one of the preceding claims,
**characterised in that**
the handle (12) and/or the shaft (11) comprises an opening (19, 20) which extends as far as the through opening (18) and is opened less than 180° in the circumferential direction.

9. Device according to one of the preceding claims,
**characterised in that**
the filament holder (15) is embodied in the form of a rotary clamp or connector (40).

## Revendications

1. Dispositif de traction de fil chirurgical comportant une poignée axiale (12) s'étendant dans le sens de la longueur du dispositif de traction de fil,
une tige (11) raccordée à la poignée axiale, une ouverture de passage (18) s'étendant dans la poignée (12) et la tige (11) dans le sens de la longueur du dispositif de traction de fil, et
un élément de serrage (14) avec un porte-fil (15) pour la fixation du fil,
dans lequel le porte-fil (15) est disposé sur un côté de la poignée axiale (12) opposé à la tige (11),
dans lequel le porte-fil (15) est raccordé à la poignée (12) par un élément de maintien (16) et l'ouverture de passage (18) s'étend dans l'élément de maintien (16), et
dans lequel le porte-fil (15) est décalé par rapport à la poignée (12) dans le sens de la longueur du dispositif de traction de fil, autrement dit n'est pas disposé latéralement à côté de la poignée (12) mais est compris dans une prolongation virtuelle de la poignée (12) dans le sens de la longueur perpendiculairement à l'axe longitudinal du dispositif de traction de fil et perpendiculairement à l'axe de rotation (y) du porte-fil (15) en vue de dessus.

2. Dispositif selon la revendication 1,
**caractérisé**
**en ce que** le porte-fil (15) est disposé radialement à l'extérieur de l'ouverture de passage (18).

3. Dispositif selon la revendication 1 ou 2,
**caractérisé**
**en ce que** le porte-fil (15) est décalé d'un intervalle (d) prédéfini perpendiculairement à l'axe longitudinal (17) de l'ouverture de passage (18).

4. Dispositif selon l'une des revendications précédentes,
**caractérisé**
**en ce que** le porte-fil (15) est configuré pour être actionné au moyen d'une clé dynamométrique.

5. Dispositif selon l'une des revendications précédentes,
**caractérisé**
**en ce que** la poignée axiale (12) présente un évidement (30) s'étendant dans le sens de la longueur, lequel s'étend depuis une zone d'épaulement (31) de la tige (11) dans le sens de la longueur vers le porte-fil (15) jusqu'à l'extrémité opposée de la poignée (12).

6. Dispositif selon la revendication 5,
**caractérisé**
**en ce que** la largeur de l'évidement (30) dans le sens de la longueur augmente en allant vers le porte-fil (15).

7. Dispositif selon la revendication 5 ou 6,
**caractérisé**
**en ce que** l'évidement (30) présente une première et une deuxième parois latérales (30a, 30b) s'étendant sensiblement dans le sens de la longueur, lesquelles se font face, et **en ce que** la deuxième paroi latérale (30b) a un angle d'inclinaison supérieur à celui de la première paroi latérale (30a) par rapport à un axe longitudinal (17) de l'ouverture de passage (18).

8. Dispositif selon l'une des revendications précédentes,
**caractérisé**
**en ce que** la poignée (12) et/ou la tige (11) présentent une ouverture (19, 20) allant jusqu'à l'ouverture de passage (18) et ouverte sur moins de 180° dans la direction circonférentielle.

9. Dispositif selon l'une des revendications précédentes,
**caractérisé**
**en ce que** le porte-fil (15) est réalisé sous la forme d'une pince tournante (40).
